# EUROPEAN PATENT SPECIFICATION

(11) **EP 4 153 063 B1**
(45) Date of publication and mention of the grant of the patent: **28.02.2024**
(21) Application number: 21731292.5
(22) Date of filing: 18.05.2021
(51) Int. Cl.: A61B 10/00, A61B 10/02, A61F 13/38

(54) **SWAB FOR TAKING BIOLOGICAL SAMPLES AND METHOD FOR MAKING SUCH A SWAB**
TUPFER ZUR ENTNAHME BIOLOGISCHER PROBEN UND VERFAHREN ZUR HERSTELLUNG EINES SOLCHEN TUPFERS
ÉCOUVILLON POUR LA PRISE D'ÉCHANTILLONS BIOLOGIQUES ET PROCÉDÉ DE FABRICATION D'UN TEL ÉCOUVILLON

(30) Priority: 20.05.2020 IT 202000011683
(43) Date of publication of application: 29.03.2023
(73) Proprietor: Vacutest Kima S.r.l., 35020 Arzergrande, Padova (IT)
(72) Inventor: CHIARIN, Renzo, 35020 Arzergrande, Padova (IT); CHIARIN, Ulisse, 35020 Arzergrande, Padova (IT)
(74) Representative: Zanettin, Gianluigi
(86) International application number: PCT/IB2021/054265
(87) International publication number: WO 2021/234564

(56) References cited:
- JP-A- 2013 224 951
- US-A1- 2005 031 828
- US-A1- 2007 282 222
- US-A1- 2020 093 467

## Description

### FIELD OF APPLICATION

The present invention relates to a swab for taking biological samples, and to a method for making such a swab.

### BACKGROUND ART

In the field of clinical and diagnostic analyses it is known to take biological samples of organic material from patients, in particular from the oral-nasal, ocular, rectal, urethral, or vaginal cavities, by means of sampling devices referred to as swabs.

A swab essentially consists of a support rod which, at a free end or tip portion thereof, is coated with a fibrous layer, consisting of synthetic fibres (e.g., rayon, polyester, polyamide) or natural fibres (e.g., cotton). The layer of fibres is made so to have hydrophilic properties so as to allow a rapid absorption of an amount of sample to be taken and tested. Generally, the fibres forming the fibrous layer are fixed to the rod by a layer of adhesive.

There are two main types of swabs on the market, which differ from each other in the manner in which the fibrous layer is made: cotton swabs and flocked swabs.

Cotton swabs are made by winding a wad of fibres around a tip portion of the support rod, on which a layer of adhesive has been previously applied.

Cotton swabs ensure a good liquid sample absorption capacity and are generally used for seeding on culture mediums to check for bacterial growth. Very often, after taking the sample, the absorbent part of the swab is stored inside a test-tube containing a gelatinous substance, referred to as transport medium, which has the task of preserving the taken sample until the analytical step. An example of such a type of device is described in European patent EP643131B1.

At the time of analysis, the release of the sample from a cotton swab occurs simply by grasping the swab rod and gently swiping the tip with the fibrous layer impregnated with liquid, for example, on a Petri dish with culture medium. In practice, the sample is spread on the dish with an operation referred to as swabbing.

Although the swabbing operation is repeated and accurate, it does not, however, allow the entire absorbed sample volume to be released since the part of the sample which has penetrated inside the volume of the wad, towards the tip, during such an operation is unable to be squeezed towards the surface and then released from the swab.

Because of this limit, on average, only 40% of the liquid sample taken can be recovered from a cotton swab for analysis. This leads to a reduction in the sensitivity of the analysis and an increase in false negatives.

This limit has been accepted for a long time also due to the absence of valid alternatives to cotton swabs.

In recent years, the market has begun to prefer liquid-type transport mediums in which the absorbent part of the swab is immersed, releasing a fraction of the sample taken. The thus contaminated liquid can be used directly for seeding or other investigations. This trend has intensified with the development of machines which perform complex operations such as the automatic seeding of liquid medium samples.

However, due to the low capacity of releasing the aforementioned sample, cotton swabs are absolutely not suitable for this type of use.

The need to obtain a greater release of the sample in the liquid medium has been satisfied with the advent of flocked swabs.

Flocked swabs are made by applying fibres by means of flocking to the tip portion of the swab support rod, as described in patent EP 1608268 B2.

The flocking process has been known for a long time in various fields: textiles, furniture, automotive, cosmetics.

Flocking consists in projecting natural or artificial fibres of small dimensions against a surface coated with adhesive.

Generally, the energy to orient and accelerate the fibres, so as to make them penetrate the adhesive layer, is provided by an electrostatic field applied between the fibre dispenser and the surface to be flocked. Alternatively, the distribution and positioning of the fibres can be obtained mechanically by means of appropriate vibrators.

The fibres used for the flocking process can have lengths ranging from a few tenths of a millimetre to a few millimetres and very low counts. They can be obtained with a physical-chemical process from polyamide, rayon, viscose, cotton fibres in an infinite variety of colours and sizes.

The fibres deposited with the electrostatic flocking technique tend to arrange themselves in an orthogonal manner with respect to the gluing surface; this arrangement of the fibres favours the absorption of the liquid sample and the subsequent release thereof into the transport medium.

As highlighted in patent EP 1608268 B2, a flocked swab ensures a release of up to 900 of the absorbed sample, much higher than that of a cotton swab, which is about 400.

However, flocking is an onerous process which requires long production times and complex plants. In particular, after applying the fibres it is necessary to wait for the glue to dry and then clean the swab of any residual fibres which have not adhered to the adhesive layer, or of particles which have detached from the adhesive layer. The cleaning is carried out by brushing, vacuuming, or blowing, for example. This further complicates the production process.

There is therefore a need in the field to make swabs with fibres arranged orthogonally to the surface of the support rod without however using the flocking technique.

JP 2013 224951 A discloses a swab according to the preamble of claim 1.

US 2005/031828 A1, US 2020/093467 A1 and US 2007/282222 A1 disclose further prior art documents.

### PRESENTATION OF THE INVENTION

Therefore, it is the object of the present invention to eliminate or at least mitigate the drawbacks of the prior art mentioned above, by providing a swab for taking biological samples which has a layer of fibres arranged orthogonally to the surface of the support rod and which can be made in an operatively simpler manner than a flocked swab.

It is a further object of the present invention to provide a swab for taking biological samples which has a biological sample absorption and release capacity which is comparable to that of a flocked swab.

### BRIEF DESCRIPTION OF THE DRAWINGS

The technical features of the invention according to the aforesaid objects may be clearly found in the contents of the claims hereinbelow and the advantages thereof will become more apparent from the following detailed description, given with reference to the accompanying drawings which show one or more embodiments merely given by way of non-limiting example, in which:
- Figures 1a, b, c, d, e show some examples of swabs according to the invention;
- figure 2 shows some examples of support rods which can be used to make a swab according to the invention;
- figure 3 shows a diagrammatic plan view of a portion of a textile substrate with fibres which can be used to make a swab according to the invention;
- figure 3a shows a diagrammatic side view of the textile substrate portion shown in Figure 3;
- figure 4 shows in sequence the modes of winding a textile substrate with fibres on a support rod in accordance with a first embodiment of the method for making swabs according to the invention;
- figure 5 shows in sequence the modes of winding a textile substrate with fibres on a support rod in accordance with a second embodiment of the method for making swabs according to the invention;
- figure 6 shows a diagrammatic sectional view of a swab according to the invention;
- figures 7 and 8 show two details of figure 6 at different enlargements; and
- figures 9 and 10 show in a diagrammatic and simplified manner the production steps of a velvet ribbon;
- figure 11 shows Table A, in which the data related to comparative tests between swabs according to the invention and flocked swabs are collected, carried out having a sample volume available for absorption equal to 50µL;
- figure 12 shows Table B, in which the data related to comparative tests between swabs according to the invention and flocked swabs are collected, carried out having a sample volume available for absorption equal to 100µL; and
- figure 13 shows Table C, in which the data related to comparative tests between swabs according to the invention and flocked swabs are collected, carried out having a sample volume available for absorption equal to 200µL.

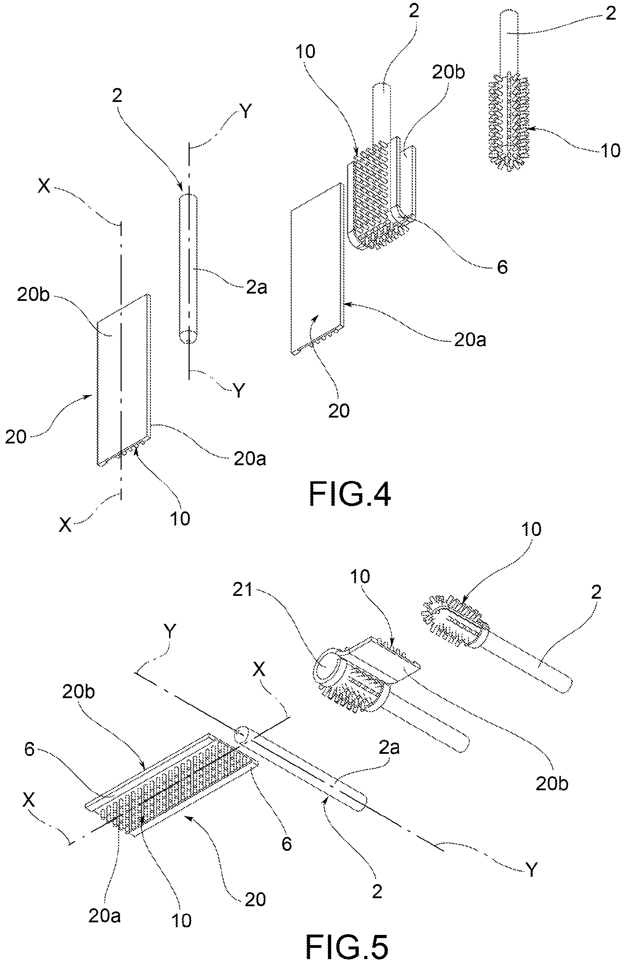

The images used in Figures 4, 5, 9 and 10 are intentionally out of scale, with details depicted not in proportion, to highlight details otherwise not graphically depictable.

### DETAILED DESCRIPTION

The present invention relates to a swab for taking biological samples, intended in particular for taking samples from the oral-nasal, ocular, rectal, urethral, or vaginal cavities of a patient. The scope of the invention is defined by independent claims 1, 11 and 12. Preferred embodiments are defined in the dependent claims.

With reference to the accompanying drawings, reference numeral 1 indicates as a whole a swab for taking biological samples according to the invention.

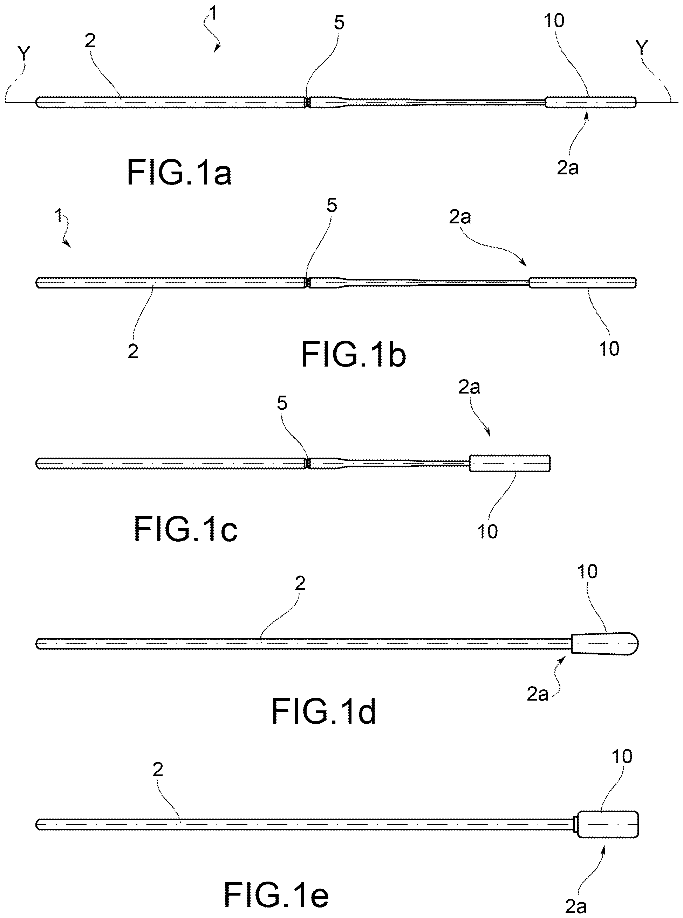

In accordance with a general embodiment of the invention, the swab 1 for taking biological samples comprises a support rod 2 having an end portion 2a coated with a layer of fibres 10. Some examples of swabs 1 are diagrammatically depicted in Figures 1a, b, c, d, e.

The support rod 2 can be of any length and shape according to the specific requirements which the swab must fulfil.

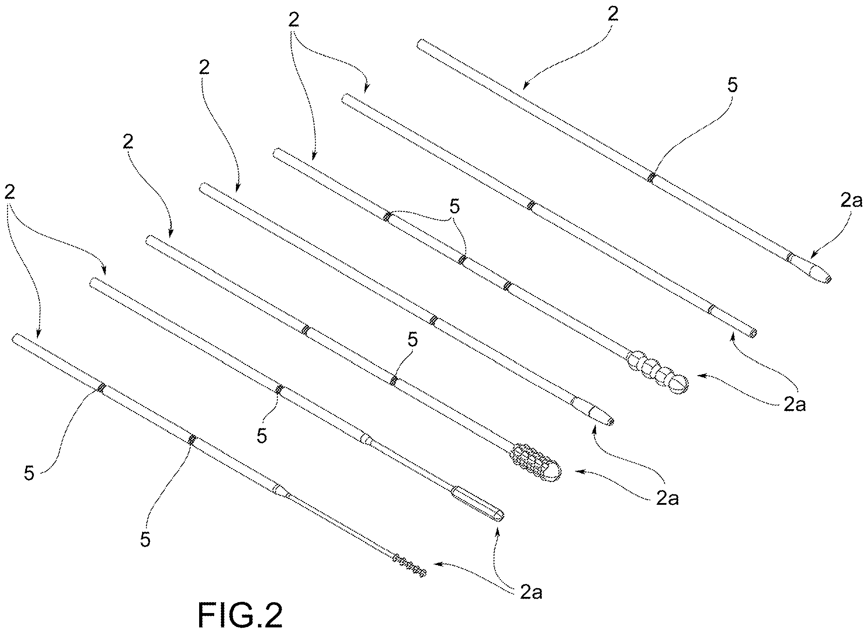

In particular, as depicted in Figure 2, the end portion 2a intended to be coated can be cylindrical, ogival in shape or have protruding appendages defining localized enlargements. In particular, the support rod 2 can be provided with one or more circumferential bottlenecks 5, included as breaking points of the rod 2, so as to separate the end portion coated with the layer of fibres 10 from the rest of the rod 2.

The rod 2 can be made of any suitable material, which gives an adequate rigidity to the rod itself to allow the operation thereof during use. Preferably, the rod 2 is made of plastic material, such as polypropylene, polyester, polyethylene, polyamide, shockproof polystyrene, etc.

Each of the fibres 10 of such a layer extends in length substantially orthogonally to the surface of the support rod 2 with a corresponding free end portion 10a.

The aforesaid layer of fibres 10 has hydrophilic properties to allow the absorption of biological samples. The hydrophilic properties of the layer of fibres 10 result from the arrangement of the fibres themselves inside the layer and from the phenomena of capillarity induced by the mass of fibres.

According to the invention, each of the aforementioned fibres 10 is mechanically anchored at the bottom portion 10b thereof to a textile substrate 20. In turn, the textile substrate 20 is fixed to a surface of the end portion 2a of the rod 2.

By virtue of the invention, and unlike traditional flocked swabs, the fibres are not deposited on the support rod directly by flocking, but by means of a textile substrate. Thereby, the formation of the layer of fibres can be carried out separately from the step of fixing such a layer to the support rod. This significantly simplifies the manufacturing process of the swab 1, completely freeing it from the flocking technique.

As already mentioned, the function of absorbing biological samples is performed by the layer of fibres 10, mainly due to capillarity phenomena between the fibres.

The amount of biological sample which can be collected from a swab 1 depends on the features of the layer of fibres 10 (fibre count, length, surface distribution density) and on the surface extension of the layer of fibres 10.

Advantageously, as a function of the features of the substrate 20, the substrate 20 itself can also partially contribute to the absorption function of the biological samples, in addition to the layer of fibres. Differently, the layer of adhesive to which the fibres are directly fixed in flocked swabs cannot contribute to the absorption of biological samples.

Preferably, each of the aforementioned fibres 10 is wedged in the weave of the textile substrate so as to be mechanically retained thereby.

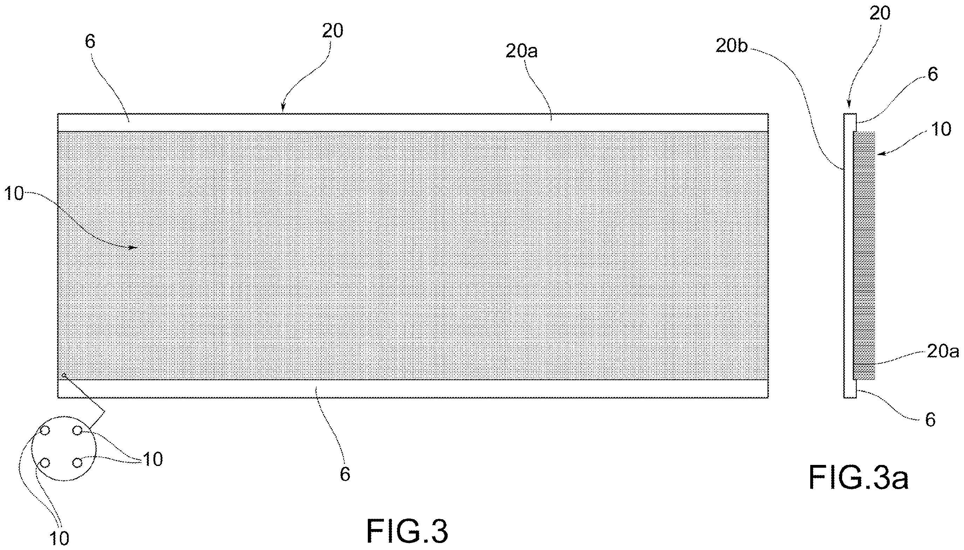

As shown in particular in Figure 3a, the following can be identified on the textile substrate 20:
- an outer face 20a, from which the fibres 10 extend and which faces the outside of the swab 1; and
- an inner face 20b, which is opposite the outer face and which adheres to the surface of the end portion 2a of the rod 2.

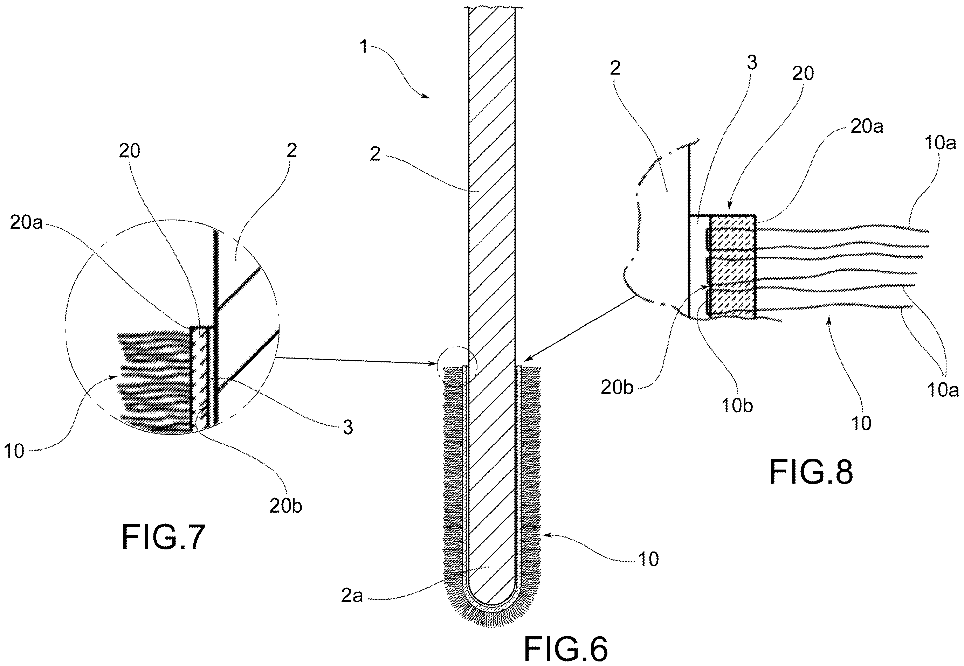

In particular, as diagrammatically shown in figure 8, each of the aforesaid fibres 10 crosses the textile substrate 20 so that:
- the respective free end portion 10a extends orthogonally from the aforesaid outer face 20a of the textile substrate 20, and
- the respective bottom portion 10b extends from the inner face 20b of the textile substrate 20 and is arranged between the inner face 20b and the surface of the end portion 2a of the rod 2.

Preferably, as diagrammatically shown in Figures 8, 9 and 10, the aforesaid fibres 10 are joined together in pairs at the respective bottom portions 10b to form a single body.

More in detail, each pair of "joined" fibres is defined by a single fibre piece passing through the substrate 20 in two different points. Thereby, the two ends of such a piece, protruding from the outer face 20a of the substrate 20 at two different points, form the free end portions 10a of two fibres 10, while the portion of the piece which is located below the substrate, adjacent to the inner face 20b of the latter, forms the bottom portions 10b of such two fibres 10.

In particular, such an arrangement of the fibres can be easily obtained by means of a simple process of sewing the fibres onto the substrate.

Advantageously, the textile substrate 20 and the fibres 10 are associated with the aforesaid end portion 2a of the rod 2 as a pre-assembled body.

In accordance with a wholly preferred embodiment of the invention, the aforesaid pre-assembled body consists of a velvet ribbon.

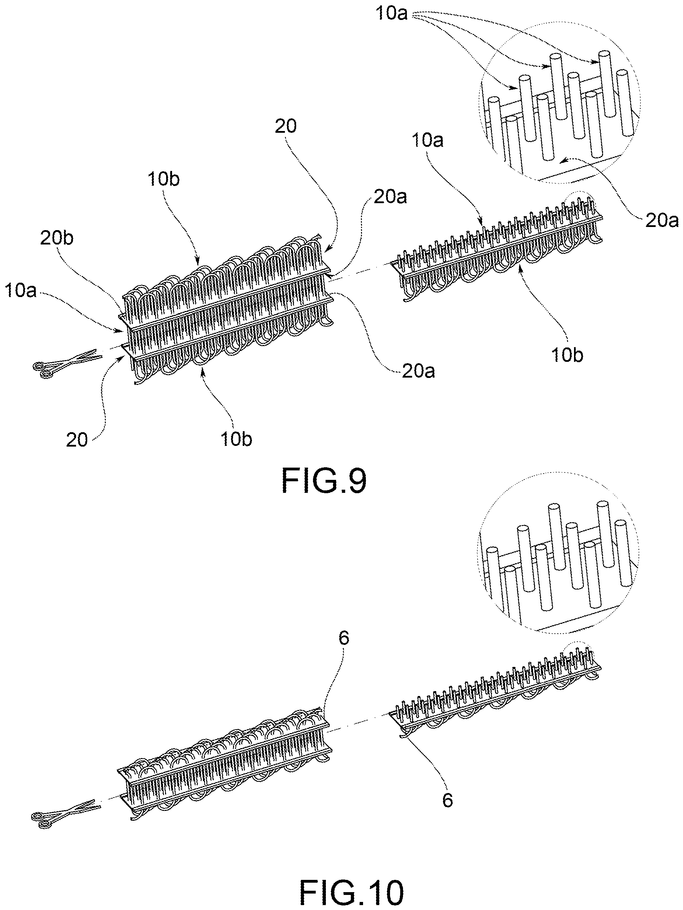

The production process of a velvet ribbon is well known per se and is diagrammatically shown in Figures 9 and 10. More in detail, two textile ribbons, preferably edged in a longitudinal direction by selvedges 6, are tightly sewn together, until the surface between the two edges of the ribbon is filled with stitches. The two ribbons are then separated from each other by cutting the sewing threads at the gap existing therebetween. The cut sewing threads separate, creating a layer of free hairs (fibres) on one face of each ribbon, which are anchored to the ribbon (textile substrate) at the bottom thereof. The density of the free hair layer depends on the density of the stitches.

Preferably, the fibres 10 extend from the textile substrate 20 outwards with substantially the same length. In other words, the layer of fibres 10 has a substantially uniform height.

Preferably, such a length is between 0.5mm and 3mm, and even more preferably between 1.0mm and 1.30mm.

Preferably, the aforementioned fibres 10 are uniformly distributed on the textile substrate 20. Even more preferably, the aforementioned fibres 10 are uniformly distributed on the textile substrate 20 with a surface density between 80 and 130 fibres/mm2.

According to the invention, the aforesaid fibres 10 have a count between 60 dtex and 90 dtex, and even more preferably between 70 dtex and 80 dtex. 1 dtex = 1 g/km.

The use of fibres within the aforesaid count ranges allows to increase the mechanical strength of the fibres themselves, reducing the risk of accidental breakage of the fibres themselves and consequent dispersion thereof during the operations of taking biological samples.

Furthermore, the use of fibres within the aforesaid count ranges allows to reduce the incidence of intertwining fibres compared to the use of fibres with lower counts.

It has been verified that the use of fibres within the aforesaid count ranges does not negatively affect the absorption capacity of the layer of fibres.

The fibres 10 can be:
- in synthetic material, preferably selected from the group consisting of rayon, polyester, polyamide, polypropylene, polyethylene, or
- in natural material, preferably selected from the group consisting of cotton and silk, or
- blends thereof.

In accordance with a preferred embodiment of the invention, the fibres 10 are made of polyamide, preferably nylon 6 or nylon 6,6.

Preferably, the aforesaid textile substrate 20 is fixed directly to the surface of the end portion 2a of the rod 2.

Even more preferably, the aforesaid textile substrate 20 is glued to the surface of the end portion 2a of the rod 2. As diagrammatically shown in Figure 7, a layer of adhesive 3 can form between the textile substrate 20 and the surface of the rod 2, which can partially penetrate the textile substrate 20. Preferably, however, the adhesive does not go beyond the textile substrate 20 so as not to invade the layer of fibres 10 extending from the outer face 20a of the substrate 20 and which come into direct contact with the patient's body in use.

Advantageously, as diagrammatically shown in Figure 8, the adhesive layer 3 can, however, come into contact with the bottom portions 10b of the fibres. This further contributes to stabilizing the fibres 10 on the swab 1, reducing the risk of accidental detachment of the fibres themselves.

Alternatively, other modes of fixing the substrate 20 to the rod 2 can be included, for example by heat or ultrasound, as a function of the material with which the substrate itself is made. However, gluing is the preferred method.

The aforesaid textile substrate 20 can be made with threads:
- in synthetic material, preferably selected from the group consisting of rayon, polyester, polyamide, polypropylene, polyethylene, or
- in natural material, preferably selected from the group consisting of cotton and silk, or
- blends thereof.

In accordance with a preferred embodiment of the invention, said textile substrate 20 consists of polyamide threads, preferably nylon 6 or nylon 6,6.

Preferably, the textile substrate 20 is made of the same material as the fibres 10, although different materials can also be used.

Preferably, the textile substrate 20 consists of threads having a count between 30 dtex and 120 dtex. The warp and weft threads can have the same count. However, it is possible to differentiate the count of the weft threads with respect to the warp threads. For example, it is possible to make a textile substrate in which the weft threads have a count of 33 dtex, while the warp threads have a count of 110 dtex.

Preferably, the aforesaid textile substrate 20 has a weight between 20 g/m2 and 600 g/m2.

Advantageously, the weight of the substrate 20 is chosen as a function of the contribution in terms of absorption of the biological samples to be obtained from the substrate 20.

The present invention also relates to a kit for taking and transporting biological samples. Such a kit comprises a test-tube containing a culture medium (preferably liquid), and a swab 1 according to the invention, and in particular as described above.

The present invention also relates to a method for making a swab 1 for taking biological samples according to the invention, and in particular as described above.

In accordance with a form of general implementation of the invention, the method comprises the following operating steps:
a) providing a support rod 2 comprising a free end portion 2a;
b) providing a textile substrate 20 comprising a first face 20a from which a plurality of fibres 10 mechanically anchored to said textile substrate 2 extends orthogonally; and
c) covering the free end portion 2a of said rod 2 with the textile substrate 20.

The covering step c) is carried out by fixing the textile substrate 20 to the rod 2 at a second face 20b of the substrate 20, opposite said first face 20a, so that the fibres 10 extend orthogonally from the surface of the free end portion 2a of the rod 2 covered by the substrate, forming a layer of fibres 10 which extends all around the aforesaid free end portion 2a.

In accordance with a preferred embodiment of the method, the textile substrate 20 provided with fibres 10 consists of a velvet ribbon.

Preferably, the aforesaid textile substrate 20 is fixed to the rod 2 by gluing by application of an adhesive to said textile substrate 20 and/or to the rod 2. The substrate 20 is adhered to the portion of the rod to be coated by interposing a layer of glue/adhesive between the substrate and the rod.

As already mentioned, other modes of fixing the substrate 20 to the rod 2 can alternatively be included, for example by heat or ultrasound, as a function of the material with which the substrate itself is made. However, gluing is the preferred method.

Preferably, the method comprises a step d) of applying a glue on the second face 20b of said textile substrate 20. The application of the glue / adhesive is carried out before the covering step c).

The adhesive/glue can be applied on the rod 2, rather than on the textile substrate. However, it is preferable to apply the adhesive on the substrate rather than on the rod 2 as this is not only operatively simpler but ensures a better and more precise fixing of the substrate on the rod.

Advantageously, the aforesaid glue/adhesive is chosen with a sufficiently high viscosity as not to completely penetrate the textile substrate 20 and thereby not be absorbed by capillarity by the fibres 10 extending from the first face 20a of the substrate 20.

Preferably, the aforesaid textile substrate 20 is ribbon-shaped having a prevailing longitudinal direction of extension X, while the free end portion 2a of the rod 2 has a prevailing extension axis Y.

In accordance with a first embodiment of the method according to the invention, diagrammatically shown in Figure 5, during the aforesaid covering step c) the textile substrate 20 (in the form of a ribbon) is wound along the aforesaid prevailing longitudinal direction of extension X on said free end portion 2a of the rod 2 around the prevailing extension axis Y until it covers all said free end portion 2a.

As a function of the height of the ribbon and the length of the free end portion 2a to be coated, the winding may require a single turn, or several turns, for example according to a spiral pattern.

Preferably, the substrate 20 is wound around said free end portion 2a so that a portion 21 of the substrate 20 itself protrudes beyond the free end of the rod 2. The portion 21 of substrate protruding beyond the free end of the rod 2 is partially cut. In particular, the selvedge of the edge is cut, and the dimensions of the protruding portion are adapted to the portion of the rod still to be covered. The residual protruding strips are then forced to close radially on the free end 2a to form a continuous surface also at the free end of the rod 2.

The winding mode described above (fig. 5) is particularly suitable for coating portions of rods with larger diameters.

In accordance with a second embodiment of the method according to the invention, diagrammatically shown in Figure 4, during the aforesaid covering step c) a portion of textile substrate 20 (in the form of a ribbon) is placed at the centre thereof on the end of the rod 2. The two symmetrical strips of the substrate are then folded on the rod 2 so as to form a cap which is then pressed so as to adhere to the rod itself.

Preferably, the excess ribbon (and in particular the selvedges of the edges) is cut along cutting lines parallel to the prevailing extension axis Y of the rod 2 and the remaining ribbon is made to adhere to the rod to obtain a continuous surface along said cutting lines.

The winding mode just described (fig. 4) is particularly suitable for coating portions of rods with smaller diameters.

In general, the method according to the invention, and in particular the two specific modes of winding the textile substrate described above, can be automated and allow to make swabs in a simple manner, making them immediately available for packaging, unlike production by flocking which requires long drying times and subsequent cleaning of excess fibres.

Comparative tests were conducted between flocked swabs and swabs made according to the invention. It was found that in many cases, with the same surface covered with fibres, the swabs according to the invention allow the absorption of a greater amount of biological sample than that of the flocked swabs. Such a result can be explained by the contribution in terms of absorption given by the textile substrate present in the swabs according to the invention.

It has also been verified that in many cases, with the same amount of sample available for absorption, the swabs according to the invention allow a higher release of the sample, which can be estimated at about 10%-15% more.

### COMPARATIVE TESTS

Comparative tests were conducted between a swab according to the invention and a flocked swab among the most widespread on the market.

The swab according to the invention had the following features: - surface coated with fibres equal to 130 mm2; - textile substrate with a weight of 170 g/m2; warp threads 110 dtex; weft threads 33 dtex; protruding fibres 78 dtex and length 1.2 mm (extension with respect to the substrate); substrate and fibres made of 100% polyamide (nylon 6). The swab according to the invention had a surface coated with fibres equivalent to that of the flocked swab.

The comparative test was carried out in two parts: in the first part the amount absorbed by the swabs was evaluated, while in the second part (immediately sequential to the first part) the amount released by the swabs was evaluated.

To evaluate the amount absorbed by the swabs, the following procedure was carried out. Deionized water was taken from a container using a pipette and deposited in a dish. Several tests were conducted by varying the amount of water available in the dish and then by varying the amount absorbed by the swabs. The use of deionized water (pure grade II water) allows the density to be dependent only on temperature (and minimally) and the absorption to not be linked to the absorbed liquid/substrate affinity. Once the drop was placed on the dish, the amount in grams deposited was measured on a thousandth scale (division 1mg). Then the swab under examination was taken and, for a time of 5 seconds, an attempt was made to absorb the maximum amount of liquid by turning the swab around the drop. Once the swab was extracted, the remaining amount was measured in grams. The amount absorbed by the swab is equal to the difference between the deposited amount and the remaining amount. Differential measurement is a measurement which also has the advantage of being free from any absolute measurement error which the scale could have.

The following procedure was carried out to evaluate the amount released by the swabs. A previously prepared piece of filter paper (50x50 mm squares) was placed on the scale and the scale was tared. Taking the swab just soaked in ionized water, it was rotated for 5 seconds around the filter paper while applying light pressure, in order to transfer the maximum amount of liquid from the swab to the filter paper. The entire surface of the filter paper was used. Also in this case, by means of differential measurement, the amount of liquid released by the swab was thus determined.

In each analysis, for each sample, two values were obtained: absorbed amount and released amount. Data analyses were then carried out on these two sets of values.

The tests were conducted on three different volumes of water available on the dish: 50µL, 100µL and 200µL. The volumes 50uL and 100µL were chosen because they correspond to the volumes of bacterial suspension which are normally inoculated in quality control tests. On the other hand, the volume 200 µL was chosen to highlight how the trend of the absorption and release cycle stabilizes at infinite volume.

As regards the time available for both absorption and release, the value of 5 seconds was chosen, a time certainly used in the general clinical use of swabs.

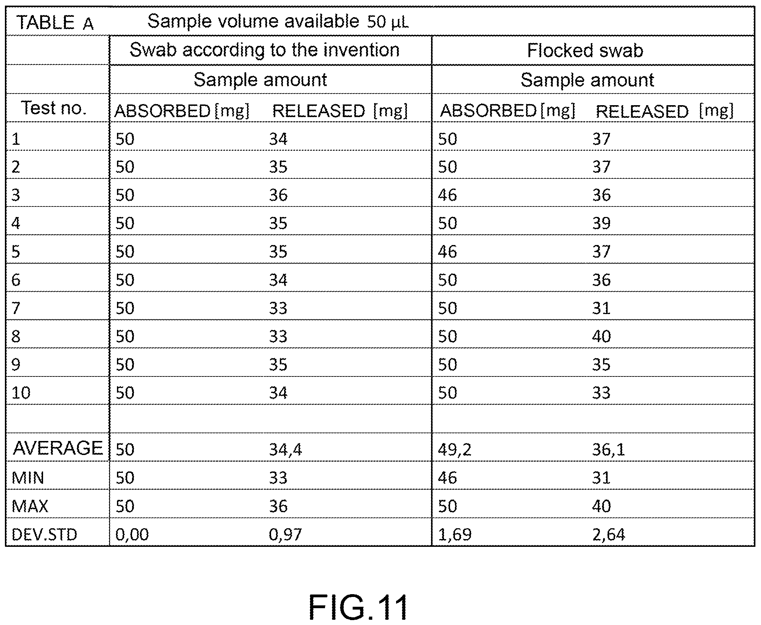

Table A, shown in Figure 11, collects the data related to the comparative tests between the swab according to the invention and the flocked swab, carried out having a volume of water available on the dish equal to 50µL.

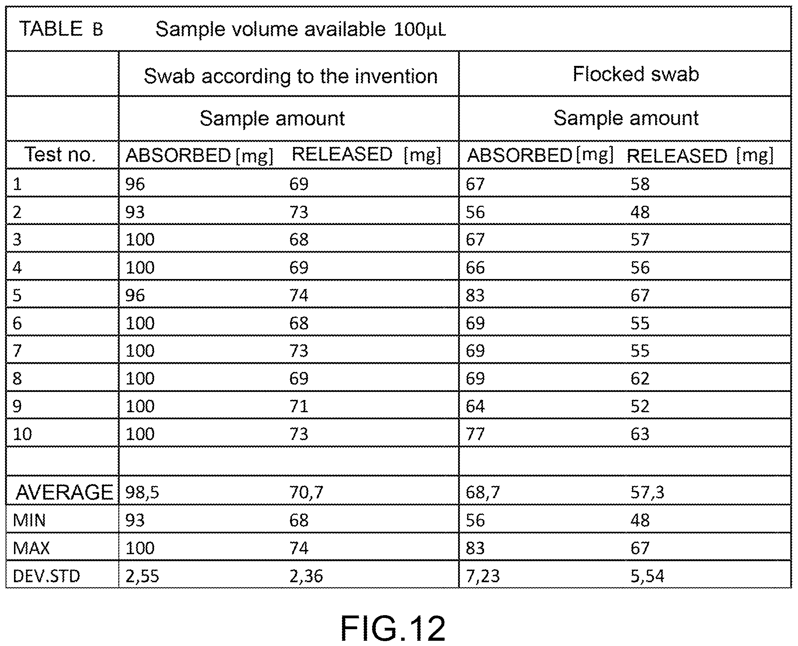

Table B, shown in Figure 12, collects the data related to the comparative tests between the swab according to the invention and the flocked swab, carried out having a volume of water available on the dish equal to 100µL.

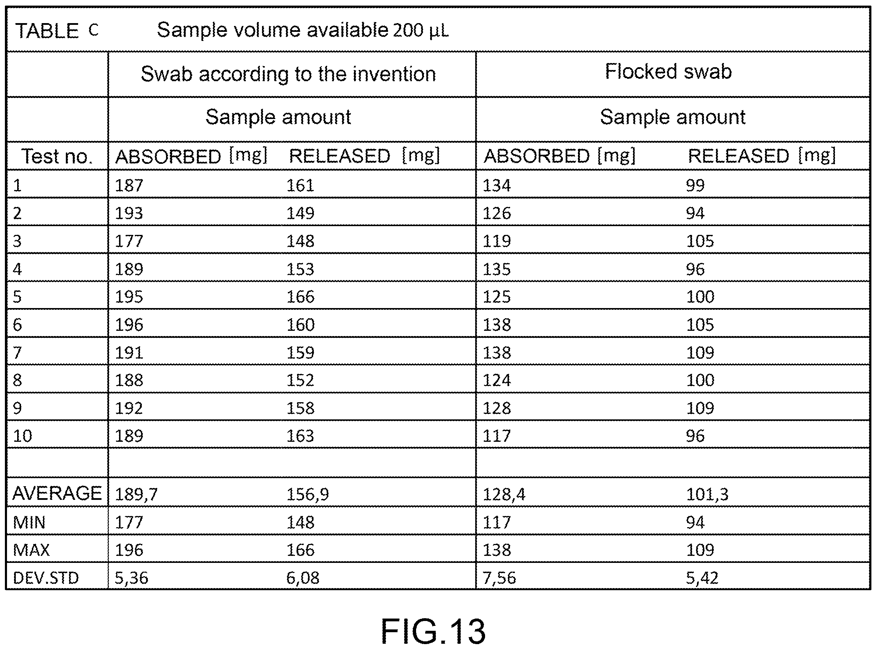

Table C, shown in Figure 13, collects the data related to the comparative tests between the swab according to the invention and the flocked swab, carried out having a volume of water available on the dish equal to 200µL.

The comparative tests conducted highlight that the swab according to the invention has features and functionality comparable to the flocked swabs available on the market, which currently represent the standard in the microbiological field, especially as regards virological analyses.

In particular, the tests highlighted a greater absorption capacity in absolute terms of the swab according to the invention with respect to flocked swabs, both as regards samples of 100µL and 200µL, and as regards samples of 50µL.

As regards the release, it can be noted that in the case of samples with a volume of 50uL the releases in absolute terms are very similar between swabs according to the invention and flocked swabs, while in the cases of 100 µL and 200µL the release in the swabs according to the invention is superior to the flocked swab.

Finally, the comparative tests demonstrated that the swab according to the invention, over a period of a few seconds (time corresponding to clinical use), absorbs a certain amount of sample, and is then able to release a large part of this absorbed amount.

It can therefore be concluded that a swab for taking biological samples according to the invention has at least a biological sample absorption and release capacity which is comparable to (if not even higher than) that of a flocked swab.

The invention allows to obtain several advantages which have been explained in the description.

The swab for taking biological samples according to the invention has a layer of fibres arranged orthogonally to the surface of the support rod and can be made in an operatively simpler manner than a flocked swab.

The swab for taking biological samples according to the invention has a biological sample absorption and release capacity which is comparable to that of a flocked swab.

Therefore, the invention thus conceived achieves the pre-set objects.

Obviously, in the practice thereof, it may also take other shapes and configurations than that disclosed above, without for this reason departing from the present scope of protection. The scope of the invention is defined by the appended claims solely.

## Claims

1. Swab for taking biological samples comprising a support rod (2) having an end portion (2a) coated with a layer of fibres (10), each fibre extending in length orthogonally to a surface of the support rod (2) with a corresponding free end portion (10a), wherein said layer of fibres (10) has hydrophilic properties to allow the absorption of biological samples, wherein each of said fibres (10) is mechanically anchored at a bottom portion (10b) to a textile substrate (20) which in turn is fixed to a surface of the end portion (2a) of said rod, **characterised in that** said fibres (10) have a count between 60 dtex and 90 dtex.

2. The swab according to claim 1, wherein each of said fibres (10) crosses said textile substrate (20) in such a way that the respective free end portion (10a) extends orthogonally from an outer face (20a) of said textile substrate (20), and the respective bottom portion (10b) extends from an inner face (20b) of said textile substrate (20), opposite said outer face (20a), and is positioned between said inner face (20b) and the surface of the end portion (2a) of said rod (2).

3. The swab according to claim 2, wherein said fibres (10) are joined together in pairs at the respective bottom portions (10b) to form a single body.

4. The swab according to any of the preceding claims, wherein said textile substrate (20) and said fibres (10) are joined to said end portion (2a) of said rod as a pre-assembled body.

5. The swab according to claim 4, wherein said pre-assembled body consists of a velvet ribbon.

6. The swab according to any of the preceding claims, wherein said fibres (10) extend from said textile substrate (20) outwards substantially with the same length, wherein preferably said length is between 0.5mm and 3mm, and even more preferably between 1.0mm and 1.30mm.

7. The swab according to any of the preceding claims, wherein said fibres (10) are evenly distributed over said textile substrate (20), preferably with a surface density between 80 and 130 fibres/mm².

8. The swab according to any of the preceding claims, wherein said textile substrate (20) is attached directly to the surface of the end portion (2a) of said rod (2).

9. The swab according to any of the preceding claims, wherein said textile substrate (20) consists of threads having a count between 30 dtex and 120 dtex.

10. The swab according to any of the preceding claims, wherein said textile substrate (20) has a weight between 20 g/m2 and 600 g/m2.

11. Kit for taking and transporting biological samples **characterized in that** it comprises a test-tube containing a culture medium, and a swab (1) according to any of the above claims.

12. Method for making a swab (1) for taking biological samples according to any of claims 1-10, said method comprising the following operating steps:
(a) providing a support rod (2) comprising a free end portion (2a);
(b) providing a textile substrate (20) comprising a first face (20a) from which a plurality of fibres (10) mechanically anchored to said textile substrate (2) extends orthogonally; and
(c) covering the free end portion (2a) of said rod (2) with said textile substrate (20) by fixing said textile substrate (20) to said rod (2) at a second face (20b) opposite said first face (20a) so that said fibres (10) extend orthogonally from the surface of the end portion (2a) of said rod (2) forming a layer of fibres (10) which extends all around said end portion (2a).

13. Method according to claim 12, wherein said textile substrate (20) with fibres (10) consists of a velvet ribbon.

14. Method according to claim 12 or 13, wherein said textile substrate (20) is fixed to said rod (2) by gluing by application of an adhesive to said textile substrate (20) and/or to said rod (2).

15. Method according to claim 14, comprising a step (d) of applying an adhesive to said second face (20b) of said textile substrate (20), before said covering step (c).

16. Method according to claim 14 or 15, wherein said adhesive is chosen with a sufficiently high viscosity as not to penetrate completely the textile substrate (20) and thereby not be absorbed by capillarity by the fibres (10) extending from the first face (20a) of said substrate (20).

17. Method according to any of the claims from 12 to 16, wherein said substrate (20) is ribbon-shaped with a prevailing longitudinal direction of extension (X) and the end portion (2a) of said rod (2) has a prevailing extension axis (Y), and wherein during said step c) of covering said substrate (20) is wound along said prevailing longitudinal direction of extension (X) on said end portion (2a) of said rod (2) around said prevailing extension axis (Y) until it covers all said end portion (2a).

18. Method according to claim 17, wherein said substrate (20) is wound around said end portion (2a) so that a portion of the substrate itself (20) protrudes beyond the free end of the rod (2) and wherein said portion of substrate protruding beyond the free end of the rod (2) is partially cut and the residual protruding strips are forced to close radially on the free end (2a) to form a continuous surface also at the free end of the rod (2).

19. Method according to any of the claims from 12 to 16, wherein said substrate (20) is ribbon-shaped with a prevailing longitudinal extension direction (X) and the end portion (2a) of said rod (2) has a prevailing extension axis (Y), and wherein, during said step c) of covering a portion of said ribbon (20) it is placed at its centre on the end of the rod (2) and the two symmetrical strips are then bent on the rod (2) so as to form a cap which is then pressed so as to adhere to the rod, wherein preferably the excess ribbon is cut along cutting lines parallel to the prevailing extension axis (Y) of the rod (2) and the remaining ribbon is made to adhere to the rod to obtain a continuous surface along said cutting lines.

## Patentansprüche

1. Ein Tupfer zur Entnahme biologischer Proben, umfassend einen Stützstab (2) mit einem Endabschnitt (2a), der mit einer Schicht von Fasern (10) beschichtet ist, wobei sich jede Faser in ihrer Länge orthogonal zu einer Oberfläche des Stützstabes (2) mit einem entsprechenden freien Endabschnitt (10a) erstreckt, wobei die genannte Schicht von Fasern (10) hydrophile Eigenschaften aufweist, um die Absorption von biologischen Proben zu ermöglichen, wobei jede der genannten Fasern (10) an einem Bodenabschnitt (10b) mechanisch an einem textilen Substrat (20) verankert ist, das seinerseits an einer Oberfläche des Endabschnitts (2a) des genannten Stabes befestigt ist, **dadurch gekennzeichnet, dass** die genannten Fasern (10) einen Wert zwischen 60 dtex und 90 dtex aufweisen.

2. Der Tupfer nach Anspruch 1, wobei jede der genannten Fasern (10) das genannte textile Substrat (20) so kreuzt, dass der jeweilige freie Endabschnitt (10a) sich orthogonal von einer Außenfläche (20a) des genannten textilen Substrats (20) aus erstreckt und der jeweilige Bodenabschnitt (10b) sich von einer Innenfläche (20b) des genannten textilen Substrats (20) aus erstreckt, die der genannten Außenfläche (20a) gegenüberliegt, und zwischen der genannten Innenfläche (20b) und der Oberfläche des Endabschnitts (2a) des genannten Stabes (2) angeordnet ist.

3. Der Tupfer nach Anspruch 2, wobei die genannten Fasern (10) paarweise an den jeweiligen Bodenabschnitten (10b) zusammengefügt sind, um einem einzigen Körper zu bilden.

4. Der Tupfer nach irgendeinem der vorstehenden Ansprüche, wobei das genannte textile Substrat (20) und die genannten Fasern (10) als vormontierter Körper an den genannten Endabschnitt (2a) des genannten Stabes angefügt sind.

5. Der Tupfer nach Anspruch 4, wobei der genannte vormontierte Körper aus einem Samtband besteht.

6. Der Tupfer nach irgendeinem der vorstehenden Ansprüche, wobei sich die genannten Fasern (10) vom genannten textilen Substrat (20) im Wesentlichen mit der gleichen Länge nach außen erstrecken, wobei vorzugsweise die genannte Länge zwischen 0,5 mm und 3 mm und noch bevorzugter zwischen 1,0 mm und 1,30 mm liegt.

7. Der Tupfer nach irgendeinem der vorstehenden Ansprüche, wobei die genannten Fasern (10) gleichmäßig über das genannte textile Substrat (20) verteilt sind, vorzugsweise mit einer Oberflächendichte zwischen 80 und 130 Fasern/mm².

8. Der Tupfer nach irgendeinem der vorstehenden Ansprüche, wobei das genannte textile Substrat (20) direkt an der Oberfläche des Endabschnitts (2a) des genannten Stabes (2) angebracht ist.

9. Der Tupfer nach irgendeinem der vorstehenden Ansprüche, wobei das genannte textile Substrat (20) aus Fäden mit einem Wert zwischen 30 dtex und 120 dtex besteht.

10. Der Tupfer nach irgendeinem der vorstehenden Ansprüche, wobei das genannte textile Substrat (20) ein Gewicht zwischen 20 g/m2 und 600 g/m2 hat.

11. Ein Kit zur Entnahme und zum Transport biologischer Proben, **dadurch gekennzeichnet, dass** es ein Teströhrchen umfasst, das ein Kulturmedium enthält, und einen Tupfer (1) nach irgendeinem der vorstehenden Ansprüche.

12. Ein Verfahren zur Herstellung eines Tupfers (1) zur Entnahme biologischer Proben nach irgendeinem der Ansprüche von 1 bis 10, wobei das genannte Verfahren die folgenden Arbeitsschritte umfasst:
(a) Bereitstellen eines Stützstabes (2), der einen freien Endabschnitt (2a) umfasst;
(b) Bereitstellen eines textilen Substrats (20), das eine erste Fläche (20a) umfasst, von der sich eine Vielzahl von Fasern (10), die mechanisch am genannten textilen Substrat (2) verankert sind, orthogonal erstreckt; und
(c) Bedecken des freien Endabschnitts (2a) des genannten Stabes (2) mit dem genannten textilen Substrat (20) durch Befestigen des genannten textilen Substrats (20) am genannten Stab (2) an einer zweiten Fläche (20b), die der genannten ersten Fläche (20a) gegenüberliegt, sodass sich die genannten Fasern (10) von der Oberfläche des Endabschnitts (2a) des genannten Stabes (2) orthogonal erstrecken und eine Schicht aus Fasern (10) bilden, die sich rundherum um den genannten Endabschnitt (2a) erstreckt.

13. Das Verfahren nach Anspruch 12, wobei das genannte textile Substrat (20) mit den Fasern (10) aus einem Samtband besteht.

14. Das Verfahren nach Anspruch 12 oder 13, wobei das genannte textile Substrat (20) am genannten Stab (2) durch Verkleben durch Auftragen eines Klebstoffs auf das genannte textile Substrat (20) und/oder auf dem genannten Stab (2) befestigt wird.

15. Das Verfahren nach Anspruch 14, umfassend einen Schritt (d) des Auftragens eines Klebstoffs auf die genannte zweite Fläche (20b) des genannten textilen Substrats (20) vor dem genannten Schritt des Bedeckens (c).

16. Das Verfahren nach Anspruch 14 oder 15, wobei der genannte Klebstoff mit einer ausreichend hohen Viskosität gewählt wird, um das textile Substrat (20) nicht vollständig zu durchdringen und dadurch nicht durch Kapillarität von den Fasern (10) absorbiert zu werden, die sich von der ersten Fläche (20a) des genannten textilen Substrats (20) aus erstrecken.

17. Das Verfahren nach irgendeinem der Ansprüche von 12 bis 16, wobei das genannte textile Substrat (20) bandförmig ist mit einer vorherrschenden Längsausdehnungsrichtung (X) und wobei der Endabschnitt (2a) des genannten Stabes (2) eine vorherrschende Ausdehnungsachse (Y) aufweist, und wobei während des genannten Schrittes c) des Bedeckens, das genannte Substrat (20) entlang der genannten vorherrschenden Längsausdehnungsrichtung (X) auf den genannten Endabschnitt (2a) des genannten Stabes (2) um die genannte vorherrschende Ausdehnungsachse (Y) gewickelt wird, bis es den gesamten genannten Endabschnitt (2a) bedeckt.

18. Das Verfahren nach Anspruch 17, wobei das genannte Substrat (20) so um den genannten Endabschnitt (2a) gewickelt wird, dass ein Abschnitt des Substrats selbst (20) über das freie Ende des Stabes (2) hinausragt, und wobei der genannte, über das freie Ende des Stabes (2) hinausragende Abschnitt des Substrats teilweise abgeschnitten wird und die verbleibenden überstehenden Streifen dazu gezwungen werden, sich radial auf dem freien Ende (2a) zu schließen, um auch am freien Ende des Stabes (2) eine durchgehende Oberfläche zu bilden.

19. Das Verfahren nach irgendeinem der Ansprüche von 12 bis 16, wobei das genannte Substrat (20) bandförmig ist mit einer vorherrschenden Längserstreckungsrichtung (X) und wobei der Endabschnitt (2a) des genannten Stabes (2) eine vorherrschende Erstreckungsachse (Y) aufweist, und wobei, während des genannten Schritts c) des Bedeckens, ein Abschnitt des genannten Bandes (20) mit seiner Mitte bzw. mittig auf das Ende des Stabes (2) gelegt wird, und wobei die beiden symmetrischen Streifen dann auf den Stab (2) so gebogen werden, dass eine Kappe gebildet wird, die dann gepresst wird, um am Stab zu haften, wobei vorzugsweise das überschüssige Band entlang von Schnittlinien parallel zur vorherrschenden Erstreckungsachse (Y) des Stabes (2) geschnitten wird, und das verbleibende Band dazu gebracht wird, am Stab zu haften, um entlang der genannten Schnittlinien eine kontinuierliche Oberfläche zu erhalten.

## Revendications

1. Écouvillon pour prélever des échantillons biologiques comprenant une tige support (2) ayant une partie d'extrémité (2a) revêtue d'une couche de fibres (10), chaque fibre s'étendant en longueur orthogonalement à une surface de la tige de support (2) avec une partie d'extrémité libre correspondante (10a), dans lequel ladite couche de fibres (10) a des propriétés hydrophiles pour permettre l'absorption d'échantillons biologiques, dans lequel chacune desdites fibres (10) est ancrée mécaniquement, au niveau d'une partie inférieure (10b), à un substrat textile (20) qui est à son tour fixé à une surface de la partie d'extrémité (2a) de ladite tige, **caractérisé en ce que** lesdites fibres (10) ont un titre compris entre 60 dtex et 90 dtex.

2. Écouvillon selon la revendication 1, dans lequel chacune desdites fibres (10) traverse ledit substrat textile (20) de telle sorte que la partie d'extrémité libre respective (10a) s'étend orthogonalement depuis une face extérieure (20a) dudit substrat textile (20), et la partie inférieure respective (10b) s'étend depuis une face intérieure (20b) dudit substrat textile (20), opposée à ladite face extérieure (20a), et est positionnée entre ladite face intérieure (20b) et la surface de la partie d'extrémité (2a) de ladite tige (2).

3. Écouvillon selon la revendication 2, dans lequel lesdites fibres (10) sont raccordées par paires au niveau des parties inférieures respectives (10b) pour former un corps unique.

4. Écouvillon selon l'une des revendications précédentes, dans lequel ledit substrat textile (20) et lesdites fibres (10) sont raccordés à ladite partie d'extrémité (2a) de ladite tige sous forme d'un corps préassemblé.

5. Écouvillon selon la revendication 4, dans lequel ledit corps préassemblé est constitué d'un ruban de velours.

6. Écouvillon selon l'une des revendications précédentes, dans lequel lesdites fibres (10) s'étendent depuis ledit substrat textile (20) vers l'extérieur sensiblement avec la même longueur, ladite longueur étant de préférence comprise entre 0,5 mm et 3 mm, et de manière encore plus préférée entre 1,0 mm et 1,30 mm.

7. Écouvillon selon l'une des revendications précédentes, dans lequel lesdites fibres (10) sont réparties uniformément sur ledit substrat textile (20), de préférence selon une densité surfacique comprise entre 80 et 130 fibres/mm².

8. Écouvillon selon l'une des revendications précédentes, dans lequel ledit substrat textile (20) est attaché directement à la surface de la partie d'extrémité (2a) de ladite tige (2).

9. Écouvillon selon l'une des revendications précédentes, dans lequel ledit substrat textile (20) est constitué de fils ayant un titre compris entre 30 dtex et 120 dtex.

10. Écouvillon selon l'une des revendications précédentes, dans lequel ledit substrat textile (20) a un grammage compris entre 20 g/m² et 600 g/m².

11. Kit de prélèvement et de transport d'échantillons biologiques, **caractérisé en ce qu'**il comprend une éprouvette contenant un milieu de culture, et un écouvillon (1) selon l'une des revendications précédentes.

12. Procédé de fabrication d'un écouvillon (1) destiné à prélever des échantillons biologiques selon l'une des revendications 1 à 10, ledit procédé comprenant les étapes opératoires suivantes consistant à :
(a) fournir une tige de support (2) comprenant une partie d'extrémité libre (2a) ;
(b) fournir un substrat textile (20) comprenant une première face (20a) depuis laquelle une pluralité de fibres (10) ancrées mécaniquement audit substrat textile (2) s'étendent orthogonalement ; et
(c) revêtir la partie d'extrémité libre (2a) de ladite tige (2) avec ledit substrat textile (20) en fixant ledit substrat textile (20) à ladite tige (2) au niveau d'une seconde face (20b), opposée à ladite première face (20a), de sorte que lesdites fibres (10) s'étendent orthogonalement depuis la surface de la partie d'extrémité (2a) de ladite tige (2) en formant une couche de fibres (10) qui s'étend tout autour de ladite partie d'extrémité (2a).

13. Procédé selon la revendication 12, dans lequel ledit substrat textile (20) comportant les fibres (10) est constitué d'un ruban de velours.

14. Procédé selon la revendication 12 ou 13, dans lequel ledit substrat textile (20) est fixé à ladite tige (2) par collage par application d'un adhésif sur ledit substrat textile (20) et/ou sur ladite tige (2).

15. Procédé selon la revendication 14, comprenant une étape (d) consistant à appliquer un adhésif sur ladite seconde face (20b) dudit substrat textile (20) avant ladite étape de revêtement (c).

16. Procédé selon la revendication 14 ou 15, dans lequel ledit adhésif est choisi avec une viscosité suffisamment élevée pour ne pas pénétrer complètement dans le substrat textile (20) et ainsi ne pas être absorbé par capillarité par les fibres (10) s'étendant depuis la première face (20a) dudit substrat (20).

17. Procédé selon l'une des revendications 12 à 16, dans lequel ledit substrat (20) est en forme de ruban ayant une direction d'extension longitudinale dominante (X) et la partie d'extrémité (2a) de ladite tige (2) a un axe d'extension dominant (Y), et dans lequel, pendant ladite étape c) de revêtement, ledit substrat (20) est enroulé le long de ladite direction d'extension longitudinale dominante (X) sur ladite partie d'extrémité (2a) de ladite tige (2) autour dudit axe d'extension dominant (Y) jusqu'à ce qu'il recouvre toute ladite partie d'extrémité (2a).

18. Procédé selon la revendication 17, dans lequel ledit substrat (20) est enroulé autour de ladite partie d'extrémité (2a) de telle sorte qu'une partie du substrat lui-même (20) fait saillie au-delà de l'extrémité libre de la tige (2), et dans lequel ladite partie de substrat faisant saillie au-delà de l'extrémité libre de la tige (2) est partiellement découpée et les bandes en saillie résiduelles sont forcées de se refermer radialement sur l'extrémité libre (2a) pour former une surface continue également au niveau de l'extrémité libre de la tige (2).

19. Procédé selon l'une des revendications 12 à 16, dans lequel ledit substrat (20) est en forme de ruban ayant une direction d'extension longitudinale dominante (X) et la partie d'extrémité (2a) de ladite tige (2) a un axe d'extension dominant (Y), et dans lequel, lors de ladite étape c) de revêtement d'une partie dudit ruban (20), celui-ci est placé au niveau de son centre sur l'extrémité de la tige (2) et les deux bandes symétriques sont ensuite pliées sur la tige (2) de manière à former un capuchon qui est ensuite comprimé de manière à adhérer à la tige, dans lequel de préférence le ruban en excès est coupé le long de lignes de découpe parallèles à l'axe d'extension dominant (Y) de la tige (2) et le ruban restant est amené à adhérer à la tige pour obtenir une surface continue le long desdites lignes de découpe.
